# EUROPEAN PATENT SPECIFICATION

(11) **EP 4 317 548 B1**
(45) Date of publication and mention of the grant of the patent: **22.10.2025**
(21) Application number: 22779626.5
(22) Date of filing: 17.02.2022
(51) Int. Cl.: C30B 29/22, C23C 14/08, C23C 14/34, C30B 23/08, H01M 8/12, H01M 8/1246, G01N 33/20, G01N 33/00, G01N 27/407, C01B 33/20, C01B 33/24, C01B 35/16, C04B 35/18, C04B 35/50, C23C 14/16, C01B 33/26, C04B 35/626, C04B 35/16

(54) **MULTILAYER BODY**
MEHRSCHICHTKÖRPER
CORPS MULTICOUCHE

(30) Priority: 31.03.2021 JP 2021061792
(43) Date of publication of application: 07.02.2024
(73) Proprietor: Mitsui Mining & Smelting Co., Ltd., Shinagawa-ku Tokyo 141-8584 (JP)
(72) Inventor: YABU, Sakiko, Ageo-shi, Saitama 362-0021 (JP); OYAMA, Tokiharu, Ageo-shi, Saitama 362-0021 (JP); MATSUO, Haruki, Ageo-shi, Saitama 362-0021 (JP); SOMA, Kentaro, Ageo-shi, Saitama 362-0021 (JP); TABIRA, Yasunori, Ageo-shi, Saitama 362-0021 (JP); IDE, Shingo, Ageo-shi, Saitama 362-0021 (JP)
(74) Representative: Novagraaf Technologies
(86) International application number: PCT/JP2022/006480
(87) International publication number: WO 2022/209399

(56) References cited:
- WO-A1-2019/235383
- CN-A- 107 709 603
- JP-A- 2011 165 424
- JP-A- 2013 064 194
- JP-A- 2017 024 931
- JP-A- 2017 024 931
- JP-A- 2018 163 753
- JP-A- 2019 073 430

## Description

### Technical Field

The present invention relates to a layered body that has an oxide portion and a substrate. The layered body according to the present invention is suitable for use as, for example, any type of sensor.

### Background Art

Oxide ion conductors are used in solid electrolytes of various types of batteries such as solid electrolyte fuel cells, ion batteries, and air batteries, as well as gas separation membranes of gas sensors, and various electrochemical devices. For example, Patent Literature 1 discloses the use of an oriented apatite-type oxide ion conductor in a solid electrolyte fuel cell or an oxygen sensor.

### Citation List

### Patent Literature

Patent Literature 1: U.S. Patent Application Publication No. 2018/183068

WO 2019/235383 A1, CN 107 709 603 A, JP 2019 073430 A and JP 2017 024931 A disclose other relevant prior art.

### Summary of Invention

In the case where a solid electrolyte is used, it is often the case that a layer body is formed by placing the solid electrolyte in the form of a thin film onto a substrate that is inert to an electrochemical reaction. In this case, by forming the solid electrolyte to be thin in thickness, the effect of reducing the electric resistance of the solid electrolyte can be obtained.

In order to cause a solid electrolyte to exhibit sufficient ion conductivity, there is a case where the solid electrolyte is heated. In this case, when the solid electrolyte constitutes a portion of a layered body as described above, a crack may occur between the substrate and the solid electrolyte in the layered body due to a difference in thermal expansion therebetween. The occurrence of a crack may cause a significant reduction in the performance and reliability of an electrochemical device that includes the layered body that contains the solid electrolyte.

Accordingly, it is an object of the present invention to provide a layered body that exhibits high ion conductivity and in which damage such as a crack is unlikely to occur.

The present invention provides A layered body as claimed in claim 1, comprising:
a substrate; and
an oxide portion containing an oxide and positioned on the substrate,

wherein the oxide that is contained in the oxide portion contains: at least two or more rare-earth elements; silicon; and oxygen,
the oxide that is contained in the oxide portion exhibits a diffraction peak derived from a (004) plane at a position of 2θ = 51.9° ± 0.9° in an X-ray diffraction pattern, and has an apatite crystal structure, and
a ratio of linear expansion coefficient of the oxide that is contained in the oxide portion in an a-axis direction relative to linear expansion coefficient of the substrate is 0.15 or more and 1.45 or less.

### Brief Description of Drawings

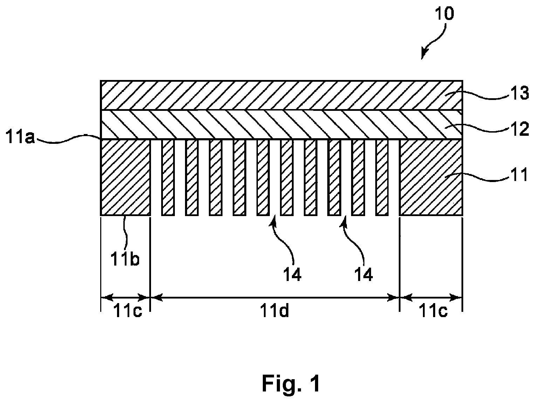

[Fig. 1] Fig. 1 is a cross-sectional view schematically showing a structure of a layered body according to an embodiment of the present invention, taken along a thickness direction of the layered body.

### Description of Embodiments

Hereinafter, the present invention will be described based on a preferred embodiment thereof. The present invention relates to a layered body that has a layered structure that includes a substrate and an oxide portion. In the layered body of the present invention, the oxide portion is placed on the substrate. According to an embodiment of the present invention, the oxide portion is placed on the substrate so as to be in direct contact with the substrate. Also, according to another embodiment of the present invention, the oxide portion is placed on the substrate indirectly on the substrate via one or more portions that are different from the oxide portion. Typical examples of the one or more portions that are different from the oxide portion include a natural oxide film formed on a silicon substrate surface, and an electrode (see Fig. 1), which will be described later.

There is no particular limitation on the shape of the substrate and the shape of the oxide portion, and various types of shapes can be used according to the specific application of the layered body of the present invention. For example, the substrate can have a plate-like body that has two opposing main surfaces. In the case where the substrate has the plate-like body, there is no particular limitation on the shape of the plate-like body when viewed in a plan view. The plate-like body can have any shape such as, for example, a rectangular shape, a polygonal shape, a circular shape, or an elliptical shape.

In the case where the plate-like body is used as the substrate, the oxide portion can be placed on at least one of the two main surfaces. In the case where the oxide portion is placed on at least one of the main surfaces, the contour of the main surface and the contour of the oxide portion can be matched when viewed in a plan view. Alternatively, the oxide portion may be placed on the main surface such that the contour of the main surface of the substrate is located outside of the contour of the oxide portion when viewed in a plan view.

The substrate is used mainly to serve as a support for the oxide portion. For this purpose, it is advantageous that the substrate has a high mechanical strength than the oxide portion.

It is also advantageous that the substrate has the property of not inhibiting the functions of the oxide portion. As will be described later, in the case where the oxide portion has oxide ion conductivity, the substrate is preferably made using a material that is inert to oxide ion conductivity.

As shown in Fig. 1, which will be described later, through holes that extend in a thickness direction of the substrate may be formed in the substrate. In general, it is convenient to use dry etching or wet etching to form through holes. Accordingly, it is advantageous to use a material suitable for these etching methods as the material for forming the substrate. From this viewpoint, examples of the material for forming the substrate include a silicon-containing material (for example, a simple substance of crystalline silicon, a silicon compound (for example, quartz, glass, or the like)), a semiconductor such as gallium arsenide, a metal such as aluminum, copper, or nickel, an alloy made using the metal, strontium titanate, a ceramic such as magnesia, and the like. Out of these materials, from the viewpoint of mass productivity and etching, it is particularly preferable to use a silicon-containing material.

Next, the oxide portion that is placed on the substrate will be described. The oxide portion is preferably made using an oxide solid electrolyte material that has oxide ion conductivity. From the viewpoint of causing the oxide portion to exhibit oxide ion conductivity, an oxide that constitutes the oxide portion is preferably made of a material that contains at least two or more rare-earth elements, silicon, and oxygen. This material is a substance in a category generally called "rare-earth silicate".

As the rare-earth elements, one or more elements selected from the group consisting of Y, La, Ce, Pr, Nd, Pm, Sm, Eu, Gd, Tb, Dy, Ho, Er, Yb, and Lu can be used. Also, the oxide that constitutes the oxide portion may contain, in addition to at least two or more rare-earth elements, silicon, and oxygen, for example, one or more elements selected from the group consisting of Be, Mg, Ca, Sr, and Ba.

The oxide that constitutes the oxide portion preferably has an apatite crystal structure. In particular, the oxide that constitutes the oxide portion preferably has an oriented apatite-type crystal structure. As used herein, the term "oriented" means that the crystal has an orientation axis. It is particularly preferable that the oxide that constitutes the oxide portion has a c-axis orientation.

When the oxide that constitutes the oxide portion is subjected to X-ray diffraction (hereinafter, also referred to as "XRD") measurement using an X-ray diffraction apparatus, characteristic diffraction peaks are observed in planes such as the (002) plane, the (004) plane, and the (006) plane. In particular, the oxide that constitutes the oxide portion is characterized by a diffraction angle (2θ) at which a diffraction peak derived from the (004) plane is observed, as compared with a conventionally known lanthanum silicate. To be specific, in an XRD pattern obtained through XRD measurement performed using a powder XRD apparatus using CuKα rays, the oxide that constitutes the oxide portion preferably exhibits a diffraction peak derived from the (004) plane at a position of 2θ = 51.9° ± 0.9° (hereinafter the diffraction peak derived from the (004) plane will be referred to as "004 diffraction peak", and likewise, a diffraction peak derived from the (002) plane and a diffraction peak derived from the (006) plane will be referred to as "002 diffraction peak" and "006 diffraction peak", respectively). In particular, the oxide that constitutes the oxide portion exhibits a 004 diffraction peak at a position of preferably 2θ = 51.9° ± 0.7°, and more preferably 51.9° ± 0.6°. Even if a plurality of peaks are observed within a range of 51.9° ± 0.9°, the 004 diffraction peak can be identified from the X-ray diffraction pattern. When the 004 diffraction peak is observed at a position of 2θ = 51.9° ± 0.9° in the oxide that constitutes the oxide portion, it can be said that the oxide that constitutes the oxide portion is crystalized, and thus has high ion conductivity.

The oxide that constitutes the oxide portion also exhibits, in addition to the 004 diffraction peak, a peak derived from the (00I) plane, where I is a positive integer, such as a 002 diffraction peak or a 006 diffraction peak. The positions of these diffraction peaks are shifted toward the high angle side unlike those of a conventionally known lanthanum silicate. Even if the oxide that constitutes the oxide portion does not have a c-axis orientation, the (00I) plane can be identified from the X-ray diffraction pattern.

As a result of studies conducted by the inventors of the present application, it was found that it is effective to adjust the composition of the oxide that constitutes the oxide portion in order for the oxide that constitutes the oxide portion to exhibit a 004 diffraction peak at a position described above. As described above, the oxide ion conductor of the present invention preferably contains at least two or more rare-earth elements, a silicon (Si) element, and an oxygen (O) element, and more preferably contains, as the rare-earth elements, a lanthanide element, and at least one or more selected from yttrium and scandium. Also, it is even more preferable that the oxide that constitutes the oxide portion contains at least a lanthanum (La) element and a yttrium (Y) element.

An oxide represented by the following formula (1) is particularly preferably used in the present invention:

A_{9.3+x-a}Yₐ[Si_{6.0-y}M_{y}]O_{26.0+z},

where A represents one or more elements selected from the group consisting of La, Ce, Pr, Nd, Pm, Sm, Eu, Gd, Tb, Dy, Ho, Er, Yb, Lu, Be, Mg, Ca, Sr, and Ba, and includes at least La,
M represents one or more elements selected from the group consisting of Mg, Al, Sc, Ti, V, Cr, Mn, Fe, Co, Ni, Ga, Ge, Zr, Ta, Nb, B, Zn, Sn, W, and Mo,
x is a number of -1.4 or more and 1.5 or less,
y is a number of 0.0 or more and 3.0 or less,
z is a number of -5.0 or more and 5.2 or less,
a is a number of 0.1 or more and 10.4 or less, and
the ratio of the number of moles of A to the number of moles of Si is 1.4 or more and 3.7 or less.

Out of the elements that can be used as A in the formula (1), La, Ce, Pr, Nd, Sm, Eu, Gd, Tb, Dy, Be, Mg, Ca, Sr, and Ba are elements that have the commonality in that they can be converted into ions that have positive electric charges and are lanthanoids or Group 2 elements that can constitute an apatite hexagonal crystal structure. Out of these elements, from the viewpoint of further increasing the oxide ion conductivity of the oxide portion, it is preferable that A represents at least one or a combination of two or more selected from the group consisting of La, Nd, Ba, Sr, Ca, and Ce, and includes at least La.

In particular, the element M in the formula (1) preferably represents one or more selected from the group consisting of B, Ge, Zn, W, Sn, and Mo. Out of these elements, from the viewpoint of a high degree of orientation and high productivity of the oxide that constitutes the oxide portion, the element M more preferably represents one or more selected from the group consisting of B, Ge, and Zn.

From the viewpoint of further increasing the degree of orientation of the oxide that constitutes the oxide portion as well as the oxide ion conductivity of the oxide portion, x in the formula (1) is preferably -1.0 or more and 1.0 or less, more preferably 0.0 or more and 0.7 or less, and even more preferably 0.4 or more and 0.7 or less.

From the viewpoint of filling the position of the Si element in the apatite crystal lattice, y in the formula (1) is preferably 0.4 or more and less than 1.0, more preferably 0.4 or more and 0.9 or less, even more preferably 0.8 or less, even much more preferably 0.7 or less, and even much more preferably 0.5 or more and 0.7 or less.

From the viewpoint of keeping electroneutrality in the apatite crystal lattice, z in the formula (1) is preferably -5.0 or more and 3.7 or less, more preferably -3.0 or more and 2.0 or less, even more preferably -2.0 or more and 1.5 or less, and even much more preferably -1.0 or more and 1.0 or less.

From the viewpoint of keeping spatial occupancy in the apatite crystal lattice, the ratio of the number of moles of A to the number of moles of Si in the formula (1), or in other words, (9.3+x-a)/(6.0-y) in the formula (1) is preferably 1.4 or more and 3.0 or less, and more preferably 1.5 or more and 2.0 or less.

Specific examples of the oxide represented by the formula (1) include, but are not limited to, La_{8.6}Y_{1.1}(Si_{5.3}B_{0.7})O_{26.7}, La_{8.0}Y_{1.7}(Si_{5.3}B_{0.7})O_{26.7}, La_{7.5}Y_{2.2}(Si_{5.3}B_{0.7})O_{26.7}, and the like.

In a preferred embodiment of the oxide that constitutes the oxide portion, the oxide that constitutes the oxide portion has a degree of orientation measured using a Lotgering method, or in other words, a Lotgering orientation factor of 0.6 or more, preferably 0.8 or more, and more preferably 0.9 or more. In order for the oxide that constitutes the oxide portion to have a Lotgering orientation factor of 0.6 or more, the oxide that constitutes the oxide portion can be produced using, for example, the method disclosed in WO 2017/018149.

In a preferred embodiment of the oxide that constitutes the oxide portion, the oxide that constitutes the oxide portion has an oxide ion conductivity at 600°C of 10⁻⁹ S/cm or more, preferably 10⁻⁸ S/cm or more, and more preferably 10⁻⁷ S/cm or more. In order for the oxide that constitutes the oxide portion to have an oxide ion conductivity at 600°C of 10⁻⁷ S/cm or more, it is preferable that the oxide that constitutes the oxide portion is configured to have a Lotgering orientation factor of 0.6 or more as described above. However, the method is not limited thereto.

In the layered body of the present invention, the linear expansion coefficient of the oxide portion and the linear expansion coefficient of the substrate are controlled. To be specific, the ratio (specifically, CT_{E}/CT_{S}, hereinafter also referred to as "thermal expansion ratio") of linear expansion coefficient CT_{E} of the oxide that constitutes the oxide portion in the a-axis direction relative to linear expansion coefficient CT_{S} of the substrate is controlled to be 0.15 or more and 1.45 or less. By controlling the linear expansion coefficients as described above, even when the layered body of the present invention is heated and cooled, it is possible to effectively suppress the occurrence of a crack in the oxide portion caused by a difference in thermal expansion between the substrate and the oxide portion. From the viewpoint of further making this advantage more prominent, the thermal expansion ratio is preferably 0.25 or more and 1.10 or less, and more preferably 0.6 or more and 0.95 or less.

Even in the case where an additional portion such as, for example, an electrode is provided between the substrate and the oxide portion, the electrode is generally thin in thickness, and thus the difference in thermal expansion between the substrate and the oxide portion becomes a problem.

A method for measuring the linear expansion coefficient CT_{S} of the substrate and the linear expansion coefficient CT_{E} of the oxide that constitutes the oxide portion in the a-axis direction will be described in Examples given later. The linear expansion coefficients CT_{S} and CT_{E} can be determined by performing measurement on the substrate and the oxide that constitutes the oxide portion before the layered body of the present invention is produced. Alternatively, the linear expansion coefficients CT_{S} and CT_{E} can be determined by performing measurement on a powder of the material that constitutes the substrate and a powder of the oxide that constitutes the oxide portion that have been taken out of the layered body of the present invention. The substrate may be mono-crystalline or poly-crystalline. In the case where the substrate is mono-crystalline, the substrate may be or may not be oriented. There is no particular limitation on the thickness of the substrate, but the thickness of the substrate is typically 10 µm or more and 1000 µm or less, preferably 100 µm or more and 650 µm or less, and even more preferably 250 µm or more and 350 µm or less.

In the case where the oxide that constitutes the oxide portion is expressed by the formula (1) given above, from the viewpoint of controlling the linear expansion coefficient of the oxide portion, it is advantageous that the oxide that constitutes the oxide portion has a space group P6₃/m. With this configuration, a crack is unlikely to occur in the oxide portion. From the viewpoint of further making this advantage more prominent, A in the formula (1) preferably includes at least La. Two crystallographically different sites (Wyckoff symbols 4f and 6h) are present in sites occupied by La atoms. In particular, it is preferable that more Y atoms are present in the Wyckoff position 4f site than in the Wyckoff position 6h site out of the sites occupied by La atoms. In order for the oxide that constitutes the oxide portion to have such a crystal structure, the oxide portion that contains Y may be calcined in a temperature range in which an apatite crystal structure can be formed.

A Rietveld refinement method is used to identify which atom site Y atoms are coordinated in. A detailed procedure will be described in Examples given later.

In the case where the substrate contains silicon, the thermal expansion ratio is preferably 0.15 or more and 1.45 or less, from the viewpoint of effectively suppressing the occurrence of a crack in the oxide portion. From this viewpoint, in the case where the substrate contains silicon, the thermal expansion ratio is more preferably 0.45 or more and 1.35 or less, and even more preferably 0.65 or more and 1.25 or less.

As the substrate that contains silicon, for example, at least one of a silicon oxide, quartz, and crystalline silicon can be used. In the case where through holes are formed in the substrate, from the viewpoint of ease of etching, the substrate preferably contains crystalline silicon.

Fig. 1 shows a cross-sectional view schematically showing a layered body according to an embodiment of the present invention, taken along a thickness direction of the layered body. A layered body 10 shown in Fig. 1 is configured such that a first electrode layer 12 and an oxide portion 13 are stacked in this order on one surface of a substrate 11.

A preferred method for producing the layered body 10 of the embodiment shown in Fig. 1 will be described below.

The method for producing the layered body 10 includes the steps of: forming an oxide portion 13; optionally forming a first electrode layer 12 before the oxide portion 13 is formed; and optionally forming a second electrode layer after the oxide portion 13 has been formed. Hereinafter, these steps will be described one by one.

First, a substrate 11 is prepared, and an oxide portion 13 is formed on one surface of the substrate 11. Any type of thin film forming means can be used to form the oxide portion 13. Specifically, the oxide portion 13 can be formed using any of physical vapor deposition (PVD) methods, such as a vapor deposition method, a sputtering method, and an ion plating method, and chemical vapor deposition (CVD) methods. Out of these various types of methods, it is preferable to use a sputtering method from the viewpoint of forming the oxide portion 13 uniformly on the substrate 11 and excellent mass productivity.

In the case where a first electrode layer 12 is formed, the step of forming the first electrode layer 12 is performed before the oxide portion 13 is formed. The first electrode layer 12 is formed on one of two surfaces of the substrate 11 on which the oxide portion 13 is to be formed. Various types of thin film forming means can be used to form the first electrode layer 12 as with the oxide portion 13. Specifically first electrode layer 12 can be formed using any of physical vapor deposition (PVD) methods, such as a vapor deposition method, a sputtering method, and an ion plating method, and chemical vapor deposition (CVD) methods. It is preferable to use a sputtering method from the viewpoint of easily forming the first electrode layer 12 that has an intended composition and excellent mass productivity.

Various types of materials can be used as a material that constitutes the first electrode layer 12. In the case where the material that constitutes the first electrode layer 12 is a cermet or the like, a calcining step of calcining the first electrode layer 12 and a step of forming holes 14 in the substrate 11 may be performed in this order during a period after the step of forming the first electrode layer 12 and before the step of forming the oxide portion 13.

In the case where the material that constitutes the first electrode layer 12 is, for example, a cermet, the calcining step of calcining the first electrode layer 12 is performed to reliably produce a calcined structure in which an ion conductive metal oxide such as samarium-doped cerium oxide (hereinafter also referred to as "SDC") is bonded by platinum or the like, or in other words, the cermet in the first electrode layer 12. From the viewpoint of reliably forming the calcined structure, the calcination temperature is preferably set to 300°C or more and 1400°C or less, more preferably 500°C or more and 1200°C or less, and even more preferably 600°C or more and 1100°C or less.

From the same viewpoint, the calcination time in the calcining step is preferably set to 1 minute or more and 20 hours or less, more preferably 10 minutes or more and 15 hours or less, even more preferably 30 minutes or more and 10 hours or less, and even much more preferably 1 hour or more and 5 hours or less.

There is no particular limitation on the calcination atmosphere used in the calcining step, and an oxygen-containing atmosphere or a reducing atmosphere may be used.

In the case where the first electrode layer 12 is a layer that contains, for example, SDC, platinum, and the like formed by performing the calcining step of calcining the first electrode layer 12, a layer composed only of SDC is formed on the surface of the substrate 11 that opposes the first electrode layer 12. Alternatively, in the case where the substrate 11 is made of silicon, an SiO₂ layer is formed on the surface of the substrate 11 that opposes the first electrode layer 12.

In the step of forming an oxide portion 13, by using a target that has an intended composition, the oxide portion 13 that has the intended composition can be formed. Calcination is performed after the oxide portion has been formed. The calcination temperature may be set to any temperature as long as the oxide portion is sufficiently crystalized, and is preferably set to 300°C or more and 1300°C or less, more preferably 500°C or more and 1200°C or less, and even more preferably 600°C or more and 1100°C or less. The calcination time is preferably set to 1 minute or more and 10 hours or less, more preferably 10 minutes or more and 5 hours or less, and even more preferably 30 minutes or more and 3 hours or less. There is no particular limitation on the calcination atmosphere used in the calcining step, and an oxygen-containing atmosphere or a reducing atmosphere can be used. Through the calcining step as described above, the crystallinity of the oxide portion 13 is increased, and high conductivity can be obtained.

Referring back to Fig. 1, the substrate 11 is a plate-like body that includes two opposing main surfaces 11a and 11b. A plurality of holes 14 are formed in the substrate 11. The holes 14 extend in a direction that intersects the surface of the substrate 11 that opposes the first electrode layer 12, or in other words, the main surface 11a shown in Fig. 1. In general, the holes 14 extend in a direction perpendicular to the surface of the substrate 11 that opposes the first electrode layer 12. The holes 14 extend passing through the two main surfaces 11a and 11b of the substrate 11, and are open on the main surfaces 11a and 11b. That is, the holes 14 are through holes. The holes 14 are formed for the purpose of improving gas supply efficiency of supplying an oxygen gas to the first electrode layer 12 in the case where the layered body 10 is used in an electrochemical element.

The holes 14 are open on the main surface 11b. The holes 14 that are open on the main surface 11b may have, for example, a circular shape. Of course, the shape of the holes 14 is not limited thereto, and may be another shape such as a triangular shape, a rectangular shape, a polygonal shape, an elliptical shape, a combination thereof, or the like. In particular, the shape of the holes 14 is preferably a circular shape or a regular polygon shape.

The holes 14 may be formed over the entire area of an opposing region where the substrate 11 and the first electrode layer 12 oppose each other, or in at least a portion of the opposing region. In the embodiment shown in Fig. 1, the holes 14 are formed in an inner region 11d of the opposing region where the substrate 11 and the first electrode layer 12 oppose each other, the inner region 11d being located on the inner side of a peripheral edge region 11c of the substrate 11.

The holes 14 that are open on the main surface 11b can be arranged regularly or randomly. There is no particular limitation on the arrangement pattern of the holes 14, and any type of arrangement pattern can be used as long as a gas can be smoothly supplied to the first electrode layer 12 via the holes 14.

The holes 14 extend linearly through the two main surfaces 11a and 11b of the substrate 11. The holes 14 have the same horizontal cross-sectional shape at any position between the two main surfaces 11a and 11b of the substrate 11. For example, in the case where the horizontal cross-sectional shape of the holes 14 is a circular shape, the holes 14 may be cylindrical spaces. Instead of this configuration, the horizontal cross-sectional shape of the holes 14 may be changed depending on the position between the two main surfaces 11a and 11b of the substrate 11. For example, the opening area of each hole 14 in an exposed surface (or in other words, the main surface 11b) of the substrate 11 may be larger than the opening area of the hole 14 in the surface (or in other words, the main surface 11a) of the substrate 11 that opposes the first electrode layer 12. That is, each of the holes 14 may be configured as, for example, a space that has a truncated cone shape.

In the method for producing the layered body 10 shown in Fig. 1, the step of forming the holes 14 may be performed after the step of forming the oxide portion 13. In this case, it is unnecessary to perform the step of forming the holes 14 in the substrate 11 during a period after completion of the step of forming the first electrode layer 12 and before the step of forming the oxide portion 13.

The thickness of the substrate 11 is preferably 10 µm or more and 1000 µm or less, more preferably 100 µm or more and 650 µm or less, and even more preferably 250 µm or more and 350 µm or less. By setting the thickness of the substrate 11 within this range, the substrate 11 sufficiently functions as a support for the first electrode layer 12, the oxide portion 13, and a later-described second electrode layer. The thickness of the substrate 11 can be measured using, for example, a vernier caliper or a digital thickness measurement device.

In the layered body 10 shown in Fig. 1, the oxide portion 13 is provided on the substrate 11 via the first electrode layer 12. In general, the oxide portion 13 has a constant thickness. For details of the oxide that constitutes the oxide portion 13, reference is made to the description given above.

From the viewpoint of effectively reducing the electric resistance of the layered body 10, the thickness of the oxide portion 13 is preferably 10 nm or more and 1000 nm or less, more preferably 30 nm or more and 500 nm or less, and even more preferably 50 nm or more and 300 nm or less. The thickness of the oxide portion 13 can be measured by observing a cross section using a stylus profilometer or an electron microscope.

In the layered body 10 shown in Fig. 1, the first electrode layer 12 is provided between the substrate 11 and the oxide portion 13. The first electrode layer 12 functions as an electrode for the oxide portion 13. As the material that constitutes the first electrode layer 12, an appropriate material can be selected according to the type of oxide that constitutes the oxide portion 13.

As the material that constitutes the first electrode layer 12, for example, a calcined structure in which an ion conductive metal oxide is bonded by platinum or the like, or in other words, a cermet or the like can be used. In addition thereto, in the case where the oxide that constitutes the oxide portion 13 is an oxide represented by the formula (1) described above, it is preferable that the first electrode layer 12 contains the following three types of materials (a) to (c) because, when the layered body 10 of the present invention 10 is used as a gas sensor, the gas sensor can operate at a lower temperature than conventional gas sensors, and the electromotive force is unlikely to vary from sensor to sensor.

Hereinafter, each of the following materials (a) to (c) will be described:
(a) one or more metals selected from the group consisting of Au, Ag, Pt, Pd, Rh, Ru, Os, and Ir;
(b) a cation conductive carbonate; and
(c) an oxide that contains: Li; and at least one selected from Ce and Sm (hereinafter also referred to as a "lithium-containing oxide").

The main purpose of using the metal (a) is to impart electron conductivity to the first electrode layer 12. Also, the metal (a) may be added to impart a catalytic activity for promoting an electrochemical reaction to the first electrode layer 12. From this viewpoint, the metal (a) is preferably one or more selected from the group consisting of Au, Ag, Pt, Pd, Rh, Ru, Os, and Ir, and more preferably one or more selected from the group consisting of Au, Ag, and Pt. It is also possible to use an electron conductive metal oxide such as zinc oxide or indium oxide.

The amount of the metal or metal oxide (a) used is preferably 20 mass% or more and 70 mass% or less relative to the total mass of the materials (a), (b), and (c) from the viewpoint of ensuring the electron conductivity of the first electrode layer 12 and obtaining high detection performance of detecting the target gas. From the viewpoint of further making this advantage more prominent, the amount of the metal (a) used is more preferably 30 mass% or more and 60 mass% or less, and even more preferably 40 mass% or more and 55 mass% or less relative to the total mass of the materials (a), (b), and (c).

The cation conductive carbonate (b) is used to impart cation conductivity to the first electrode layer 12. The cation may be, for example, an alkali metal ion such as a lithium ion or a sodium ion. From this viewpoint, the cation conductive carbonate is preferably an alkali metal salt of carbonic acid. For example, the cation conductive carbonate is preferably lithium carbonate (Li₂CO₃).

The amount of the cation conductive carbonate (b) used relative to the total mass of the materials (a), (b), and (c) is preferably 5 mass% or more and 55 mass% or less, from the viewpoint of efficiently forming a three-phase interface in the first electrode layer 12 and accurately detecting carbon dioxide in the target atmosphere. From the viewpoint of further making this advantage more prominent, the amount of the cation conductive carbonate (b) used is more preferably 7 mass% or more and 50 mass% or less, and even more preferably 10 mass% or more and 40 mass% or less relative to the total mass of the materials (a), (b), and (c).

The lithium-containing oxide (c) is an oxide that contains: Li; and at least one selected from Ce and Sm, and functions to assist conduction of anions that conduct in the oxide portion 13 and cations that conduct in the cation conductive carbonate. The lithium-containing oxide (c) may be a cation/anion conductor that can conduct both cations and anions. In the case where, for example, the oxide portion 13 has oxide ion conductivity, and the cation conductive carbonate has lithium ion conductivity, the lithium-containing oxide (c) may have both oxide ion conductivity and lithium ion conductivity.

As the material (c), for example, Li₂LnO₃, where Ln represents at least one rare-earth element, Li₂ZrO₃, Li₆Zr₃O₇ or the like can be used. Examples of Li₂LnO₃ include Li₂CeO₃, Li₂CeₓSm_{y}O₃, where x and y are positive numbers and satisfy x + y = 1, and the like. Alternatively, the material (c) may be a mixture of a lithium oxide and an oxide that contains: Li; and at least one selected from Ce and Sm. These materials are used preferably in the form of particles.

The amount of the lithium-containing oxide (c) used is preferably 10 mass% or more and 60 mass% or less relative to the total mass of the materials (a), (b), and (c) from the viewpoint of efficiently forming a three-phase interface in the first electrode layer 12 and accurately detecting a gas in the target atmosphere. From the viewpoint of further making this advantage more prominent, the amount of the lithium-containing oxide (c) used is more preferably 20 mass% or more and 50 mass% or less, and even more preferably 30 mass% or more and 40 mass% or less relative to the total mass of the materials (a), (b), and (c).

In the first electrode layer 12 that contains the materials (a), (b), and (c), it is preferable that the materials (a), (b), and (c) are uniformly mixed. When the materials (a), (b), and (c) are in a uniformly mixed state, the contact areas between the materials (a), (b), and (c) increase, and the interface resistance decreases. As a result, when the layered body of the present invention 10 is used as a gas sensor, the gas sensor can easily operate at an even lower temperature. Moreover, because the materials (a), (b), and (c) are in a mixed state, and thus the electromotive force is unlikely to be dependent on the thickness of the first electrode layer 12, as a result of which, it is possible to obtain an advantage in that the electromotive force is unlikely to vary from sensor to sensor.

In the layered body 10 of the embodiment shown in Fig. 1, a second electrode layer (not shown) may be provided on a surface of the oxide portion 13 that is opposite to the surface of the oxide portion 13 that opposes the first electrode layer 12. By providing the second electrode layer, an additional function can be imparted to the layered body 10. There is no particular limitation on the type of material that constitutes the second electrode layer as long as it functions as an electrode for the oxide portion 13. As the material that constitutes the second electrode layer, any material can be selected according to the application of the electrochemical element. For example, the second electrode layer preferably contains a platinum group element. Examples of the platinum group element include platinum, ruthenium, rhodium, palladium, osmium, and iridium. These elements can be used alone or in a combination of two or more. Also, a cermet that contains a platinum group element may also be used as the second electrode layer. Furthermore, the same type of material as that of the first electrode layer may also be used as the second electrode layer.

In the case where the layered body 10 includes a second electrode, the second electrode layer can be formed after the step of forming the oxide portion 13. In this case, the calcining step may be performed after completion of the step of forming the oxide portion 13, and also again after completion of the step of forming the second electrode layer. However, from the viewpoint of simplifying the process, the calcining step is preferably performed after completion of the step of forming the oxide portion 13 and the step of forming the second electrode layer, without performing the calcining step after completion of the step of forming the oxide portion 13.

Likewise, the calcining step after the step of forming the first electrode layer is preferably performed after completion of the step of forming the oxide portion 13 and the step of forming the second electrode layer.

The second electrode layer can also be produced using a paste, instead of using the sputtering method described above. Specifically, the second electrode layer (not shown) can be preferably formed by preparing a paste by mixing the materials (a), (b), and (c) described above at a predetermined mixing ratio and then adding an organic solvent to the mixture, applying the paste onto a surface of the oxide portion 13 to form a coating film, and calcining the coating film. The calcination temperature is preferably set to 300°C or more and 1400°C or less, more preferably 500°C or more and 1200°C or less, and even more preferably 600°C or more and 1100°C or less. The calcination time is preferably set to 1 minute or more and 20 hours or less, more preferably 10 minutes or more and 15 hours or less, even more preferably 30 minutes or more and 10 hours or less, and most preferably 1 hour or more and 5 hours or less.

In the case of preparing the paste, the proportion of the material (a) relative to the total amount of the materials (a), (b), and (c) is preferably 20 mass% or more and 70 mass% or less, more preferably 30 mass% or more and 60 mass% or less, and even more preferably 40 mass% or more and 55 mass% or less.

The proportion of the material (b) relative to the total amount of the materials (a), (b), and (c) is preferably 5 mass% or more and 55 mass% or less, more preferably 7 mass% or more and 40 mass% or less, and even more preferably 10 mass% or more and 30 mass% or less.

The proportion of the material (c) relative to the total amount of the materials (a), (b), and (c) is preferably 10 mass% or more and 60 mass% or less, more preferably 15 mass% or more and 50 mass% or less, and even more preferably 20 mass% or more and 40 mass% or less.

Also, in the case of forming the second electrode layer, the calcining step is performed after completion of the step of forming the second electrode layer, and thereafter holes 14 may be formed in the substrate 11. In this case, it is unnecessary to perform the hole forming step during a period after completion of the step of forming the first electrode layer 12 and before the step of forming the oxide portion 13.

The layered body of the present invention is preferably used as any type of gas sensor that utilizes an electrochemical reaction. For example, the layered body 10 shown in Fig. 1 can be used as a carbon dioxide sensor or an oxygen sensor. Alternatively, the layered body of the present invention can be preferably used as a solid electrolyte membrane of a solid-state oxide fuel cell.

In the case where the layered body of the present invention is used as, for example, a carbon dioxide sensor, when the layered body 10 shown in Fig. 1 is placed in a gas phase that contains carbon dioxide (for example, ambient air, an exhaust gas of an internal combustion engine, or the like), a reaction (see a formula (A) given below) takes place at a three-phase interface where the gas phase and the first electrode layer 12 are in contact with each other according to the concentration of carbon dioxide, and an equilibrium state is obtained. On the other hand, on the second electrode layer (not shown) side, a reaction represented by a formula (B) given below proceeds in response to the reaction represented by the formula (A). That is, through the above-described mechanism, an electromotive force is generated between the first electrode layer 12 and the second electrode layer. This electromotive force varies according to the concentration of carbon dioxide in the gas phase. Accordingly, by utilizing the electromotive force, it is possible to detect carbon dioxide or measure the concentration of carbon dioxide.
[Chem. 1]

2Li⁺+CO₂+(1/2)O₂+2e⁻⇄Li₂CO₃ (A)

(1/2)O₂+2e⁻⇄ O²⁻ (B)

Up to here, the present invention was described based on a preferred embodiment thereof. However, the present invention is not limited to the embodiment given above. For example, in the embodiment shown in Fig. 1, one or more intermediate layers may be formed between the oxide portion 13 and the first electrode layer 12, for the purpose of imparting an additional function to the layered body 10. Likewise, one or more intermediate layers may be formed between the oxide portion 13 and the second electrode layer (not shown).

### Examples

Hereinafter, the present invention will be described in further detail based on examples. However, the scope of the present invention is not limited to the examples given below. Unless otherwise stated, the percent sign "%" used herein means "mass%".

### Example 1

### (1) Formation of First Electrode Layer

A 300 µm-thick substrate made of silicon with a crystal orientation of <100> was prepared. The substrate had a linear expansion coefficient CT_{S} shown in Table 2 given below.

A first electrode layer with a thickness of 300 nm was formed on one surface of the substrate by performing a sputtering method. In the sputtering method, a 2 inch size platinum target and a 4 inch size SDC target were used. The first electrode layer was formed by performing a co-sputtering method in which electric power is simultaneously supplied to the two targets. DC sputtering was used for the platinum target, and RF sputtering was used for the SDC target. The flow rate of argon gas was set to 50 sccm, and the pressure of argon gas was set to 4 Pa. 200 W electric power was supplied to each target, and sputtering was performed at room temperature. The obtained first electrode layer was porous and had a bicontinuous structure in which SDC was bonded by platinum. The first electrode layer contained 12 volume% of SDC and 88 volume% of platinum.

### (2) Production of Sputtering Target for Forming Oxide Portion

La₂O₃ and SiO₂ were combined at a mass ratio of La₂O₃:SiO₂ = 80:20. Then, ethanol was added thereto and they were mixed using a ball mill. The resulting mixture was dried and milled using a mortar, and then calcined at 1650°C in an ambient atmosphere for 3 hours using a platinum crucible. Next, ethanol was added to the calcined product, and the calcined product was milled using a planetary ball mill to obtain a first preliminary calcined product powder.

Separately from the above-described operation, La₂O₃, SiO₂, and Y₂O₃ were combined at a mass ratio of La₂O₃:SiO₂:Y₂O₃ = 65:20:15. Then, ethanol was added thereto and they were mixed using a ball mill. The resulting mixture was dried and milled using a mortar, and then calcined at 1650°C in an ambient atmosphere for 3 hours using a platinum crucible. Next, ethanol was added to the calcined product, and the calcined product was milled using a planetary ball mill to obtain a second preliminary calcined product powder.

Ethanol was added to a mixture of the first preliminary calcined product powder and the second preliminary calcined product powder, and they were mixed using a ball mill. The mixing ratio of the first preliminary calcined product powder and the second preliminary calcined product powder was set such that the ratio of La and Y in an intended oxide portion was as shown in Table 2 given below. The obtained mixed powder was dispersed in ethanol to prepare a slurry. The slurry was poured into a mold. After ethanol was removed, the slurry was dried at 120°C for 4 hours to obtain a molded body. The molded body was heated and deashed at 800°C for 3 hours, and then calcined at 1620°C for 5 hours to obtain a calcined product. The calcined product was cut into a disc shape with a diameter of 100 mm and a thickness of 6 mm, and the surface of the disc was polished to obtain a sputtering target material. This target material had a composition shown in Table 2 given below. This target material was bonded to a baking plate to obtain a sputtering target.

### (3) Formation of Oxide Portion

An oxide portion with a thickness of 300 nm was formed on a surface of the first electrode layer obtained in (1) described above by performing RF sputtering using the sputtering target obtained in (2) described above. The flow rate of argon gas was set to 50 sccm, and the pressure of argon gas was set to 0.5 Pa. 200 W electric power was used, and sputtering was performed at room temperature.

### (4) Formation of Second Electrode Layer

A second electrode layer with a diameter φ of 1 mm was formed on a surface of the oxide portion formed in (3) described above using a metal mask under the same conditions as those used to form the first electrode layer. A layered body formed in this way was calcined at 900°C in an ambient atmosphere for 1 hour.

### Example 2

In this example, a layered body was obtained in the same manner as in Example 1, except that the oxide portion was formed directly on the silicon substrate without forming the electrode layers.

### Examples 3 and 4

Layered bodies were obtained in the same manner as in Example 2, except that a substrate made of Al₂O₃ with a plane orientation of (0001) was used in Example 3, a substrate made of SrTiO₃ with a plane orientation of (100) was used in Example 4, each of these substrates had a linear expansion coefficient CT_{S} shown in Table 2 given below, and the calcination temperature was set to 950°C.

### Example 5

A layered body was obtained in the same manner as in Example 2, except that the composition of the oxide portion was changed to a composition shown in Table 2 by changing the composition ratio of raw materials of the second preliminary calcined product powder to La₂O₃:SiO₂:Y₂O ₃ = 54:21:25 when forming the oxide portion, and the calcination temperature was set to 950°C.

### Comparative Example 1

A layered body was obtained in the same manner as in Example 1, except that the composition of the oxide portion was changed to a composition shown in Table 2 by changing the composition ratio of raw materials of the second preliminary calcined product powder to La₂O₃:SiO₂ = 80:20 when forming the oxide portion, and yttrium was not contained in the oxide portion.

### Comparative Example 2

A layered body was obtained in the same manner as in Example 2, except that the composition of the oxide portion was changed to a composition shown in Table 2, and yttrium was not contained in the oxide portion.

### Evaluation

The crystal structure, the 004 diffraction peak position, the space group, and the linear expansion coefficient of the oxide portion of each of the layered bodies obtained in Examples and Comparative Examples were measured using methods described below. The results are shown in Table 2.

Also, for each of the layered bodies obtained in Examples and Comparative Examples, whether a crack has occurred after heating and cracking load were determined through measurement performed using methods described below. Furthermore, for each of the layered bodies obtained in Example 1 and Comparative Example 1, conductivity was measured using a method described below. The results are shown in Table 2.

### Determination of Crystal Structure, 004 Diffraction Peak Position, and Space Group of Oxide Portion

Measurement was performed in a range of 2θ = 10° to 80° using RINT-TTRIII available from Rigaku as an XRD measurement apparatus and CuKα rays (λ = 1.5418 Å) as a ray source. The apatite crystal structure was confirmed at the 004 diffraction peak at the position of 2θ = 51.9° ± 0.9° in an XRD pattern.

### Determination of Linear Expansion Coefficient of Oxide Portion

The linear expansion coefficient of the oxide portion was measured by performing XRD analysis while changing the temperature.

Specifically, first, calcined product powders of different compositions were produced in the same manner as in (2) in Example 1. The ratio La₂O₃:SiO₂:Y₂O₃ was set to 80:20:0 (Comparative Examples 1 and 2), 65:20:15 (Examples 1 to 4), and 54:21:25 (Example 5). To each of the calcined product powders, a α-Al₂O₃ powder was added as an internal standard at a mass ratio of calcined product powder:α-Al ₂O₃ = 75:25, and they were mixed in a mortar for 5 minutes.

Smart Lab available from Rigaku was used as an XRD measurement apparatus. Also, DHS 1100 available from ANTON PAAR was used as a heating stage. A graphite heat shield was used as the heat shield of the heating stage. The measurement conditions were set as follows.
Ray source: CuKα rays (λ = 1.5418 Å)
X-ray tube voltage: 40 kV
X-ray tube current: 30 mA
Scanning method: 2θ/θ
Measurement range 2θ = 15° to 80°
Sampling width = 0.02°
Scanning speed: 1.5°min.
Measurement temperature: 30°C, 100°C, 300°C, 500°C, 700°C, and 900°C.

The lattice constant of the calcined product powder was determined by analyzing the obtained XRD spectrum using analysis software PDXL2 available from Rigaku. After correcting a diffraction angle error caused by an apparatus error due to heating from a diffraction peak derived from α-Al₂O₃ that was added as an internal standard, peak fitting was performed in which a diffraction peak of the calcined product powder was fitted using a WPPF method, and lattice constant refinement was performed in a range of 30° to 80° to obtain lattice constants in the a-axis and the c-axis at each temperature. The obtained lattice constants were plotted against temperature, and approximated with a straight line using a minimum square root method to obtain a slope (Å/K) of the straight line. A value obtained by dividing the slope (Å/K) by a lattice constant obtained at a temperature of 30°C was defined as the linear expansion coefficient (10⁻⁶/K) of the calcined product powder. The results are as shown in Table 2.

### Rietveld Refinement Method

In order to confirm in which atom site Y atoms are coordinated in the apatite crystal structure of the oxide portion, XRD spectra were acquired and analyzed based on a Rietveld refinement method. Specifically, profile fitting was performed using a program RIETAN-FP (version 2.8.3), and the atom positions of La2 species, Y1 species, Si1 species, and O4 species as well as the anisotropic temperature factors thereof were refined.

As measurement samples, calcined product powders with in which the mass ratio of La₂O₃:SiO₂:Y₂O₃ was 80:20:0 and 65:20:15 were produced in the same manner as in (2) in Example 1. To acquire XRD spectra, Smart Lab available from Rigaku was used as an XRD measurement apparatus. The measurement conditions were set as follows.
Ray source: CuKα rays (λ = 1.5418 Å)
X-ray tube voltage: 45 kV
X-ray tube current: 200 mA
Scanning method: 2θ/θ
Measurement range 2θ = 5° to 140°
Sampling width = 0.01°
Scanning speed: 0.4°/min.

In the Rietveld refinement method, a split-type pseudo Voigt function was used as the profile function and fitted to the actually measured values of XRD patterns based on a minimum square root method together with zero point correction, background, (001) orientation parameter, and lattice constant. As sites occupied by La atoms, two crystallographically different sites (Wyckoff symbols 4f and 6h) are present. As a result of detailed investigation, a reduction in electron density was indicated as compared with the case where only the 4f site is occupied by 100% La atoms. This means that the occupancy of La atoms in this site is less than 100%, or in other words, it means either one or both of the following: La atoms are deficient; and Y atoms with a less number of electrons than that of La atoms substitute a portion of La atoms. From the chemical composition described above, the abundance ratio of La atoms and Y atoms takes a constant value, and thus the ratio of Y atoms can be automatically determined based on the ratio of La atoms present in each of the 4f site and the 6h site. Specifically, the occupancy of Y atoms in the 4f site can be determined based on [the occupancy of La atoms in the 0.17647 * 4f site + the occupancy of La atoms in the 0.26471 * 6h site]. In the analysis, the two occupancies of La atoms were each independently refined. According to the calculation, the atom occupancy may take a negative value or a value greater than 100%. However, considering the actual crystal structure, this is clearly unreasonable. However, the results of analysis performed on the layered bodies obtained in Examples 1 to 4 were as shown in Table 1 given below. Specifically, the total occupancy of La atoms and Y atoms in the 4f site was 88%, and the occupancy of La atoms in the 6h site was 97%, which were positive and 100% or less, and thus reasonable. That is, the reliability of the analysis is high.

**[Table 1]**

| Site | Atom | Occupancy (%) |
|---|---|---|
| 4f | La | 53 |
| 4f | Y | 35 |
| 6h | La | 97 |

### Determination of Whether Crack Has Occurred after Heating

For each layered body, a thin film was observed at an incident voltage of 5 kV and a magnification of 5000 times and 10000 times using a JSM-7900F scanning electron microscope (SEM) available from JEOL Ltd. at room temperature to confirm whether a crack had occurred.

### Cracking Load

Each of the layered bodies obtained in Examples 1 to 4 and Comparative Examples 1 and 2 was subjected to a nano-indentation test to measure cracking load. As the measurement apparatus, TI preminer Multi Scale available from HYSITRON was used. Measurement was performed using a Berkovich indenter by setting the maximum load to 1000 mN, the load time to 5 seconds, the hold time at maximum load to 2 seconds, and the unload time to 5 seconds. When the measurement target is cracked, the load displacement curve exhibits a slight bend. This bend is defined as "cracking load".

### Conductivity

Each of the layered bodies obtained in Example 1 and Comparative Example 1 was subjected to electrochemical measurement evaluation. To be specific, measurement was performed based on an alternating current impedance method using SI 1260 available from Solartron, Inc. The measurement was performed at 600°C using a frequency from 1 MHz to 1 kHz at a measurement amplitude of 30 mV. Nyquist plot was performed using the obtained measurement result, and then, a value at an intersection with the real axis when arc fitting was performed in the frequency range was evaluated as resistance value. Based on this resistance value, conductivity was output from the fact that the electrode area of the layered body was φ 1 mm, and the electrolyte thickness was 300 nm.

**[Table 2]**

| | Substrate | | Oxide portion | | | | | Layered body | | | |
|---|---|---|---|---|---|---|---|---|---|---|---|
| | Type | Linear expansi on coefficie nt CT_{S} (ppm) | Compositio n | 004 diffraction peak position (degree) | Crystal structu re | Linear expansion coefficient CT_{E} in a-axis direction (ppm) | Spac e group | Linear expansi on coefficie nt ratio CT_{E}/CT s | Occurren ce of crack | Cracki ng load (mN) | Conductivit y (S/cm) |
| Exampl e 1 | Silico n | 3.9 | La₇Y_{2.33}Si₆ O₂₆ | 51.996 | Apatite | 2.98 | P6₃/m | 0.76 | Not observed | 870 | 2.3 × 10⁻⁷ |
| Exampl e 2 | Silico n | 3.9 | La₇Y_{2.33}Si₆ O₂₆ | 51.921 | Apatite | 2.98 | P6₃/m | 0.76 | Not observed | 870 | - |
| Exampl e 3 | Al₂O₃ | 6.7 | La₇Y_{2.33}Si₆ O₂₆ | 51.814 | Apatite | 2.98 | P6₃/m | 0.44 | Not observed | 870 | - |
| Exampl e 4 | SrTiO 3 | 11.1 | La₇Y_{2.33}Si₆ O₂₆ | 51.673 | Apatite | 2.98 | P6₃/m | 0.27 | Not observed | 870 | - |
| Exampl e 5 | Silico n | 3.9 | La_{5.55}Y_{3.78} Si₆O₂₆ | 52.796 | Apatite | 4.49 | P6₃/m | 1.15 | Not observed | 770 | - |
| Comp. Ex. 1 | Silicon | 3.9 | La_{9.33}Si₆O₂6 | 50.974 | Apatite | 5.81 | P6₃/m | 1.49 | Observed | 20 | 1.6 × 10⁻⁶ |
| Comp. Ex. 2 | Silico n | 3.9 | La_{9.33}Si₆O₂ 6 | 51.086 | Apatite | 5.81 | P6₃/m | 1.49 | Observed | 20 | - |

As is clear from the results shown in Table 2, it can be seen that in the layered bodies obtained in Examples, a crack caused due to heating was not observed.

In contrast, in the layered bodies obtained in Comparative Examples, a crack caused due to heating was observed.

### Industrial Applicability

The layered body according to the present invention is a layered body that exhibits ion conductivity and in which the occurrence of damage such as a crack is suppressed.

## Claims

1. A layered body comprising:
- a substrate (11); and
- an oxide portion (13) containing an oxide and positioned on the substrate,
wherein the oxide that is contained in the oxide portion contains: at least two or more rare-earth elements; silicon; and oxygen,
the oxide that is contained in the oxide portion has an apatite crystal structure,
**characterized in that**
the oxide that is contained in the oxide portion exhibits a diffraction peak derived from a (004) plane at a position of 2θ = 51.9° ± 0.9° in an X-ray diffraction pattern, and
a ratio of linear expansion coefficient of the oxide that is contained in the oxide portion in an a-axis direction relative to linear expansion coefficient of the substrate is 0.15 or more and 1.45 or less.

2. The layered body according to claim 1, wherein the substrate contains silicon.

3. The layered body according to claim 2, wherein the substrate contains at least one selected from the group consisting of an oxide of silicon and crystalline silicon.

4. The layered body according to any one of claims 1 to 3, wherein the oxide that is contained in the oxide portion contains: a lanthanide element; and at least one or more selected from yttrium and scandium.

5. The layered body according to any one of claims 1 to 4,
wherein the oxide that is contained in the oxide portion is represented by a formula (1): A_{9.3+x-a}Yₐ[Si_{6.0-y}M_{y}]O_{26.0+z},
where A represents one or more elements selected from the group consisting of La, Ce, Pr, Nd, Pm, Sm, Eu, Gd, Tb, Dy, Ho, Er, Yb, Lu, Be, Mg, Ca, Sr, and Ba, and includes at least La,
M represents one or more elements selected from the group consisting of Mg, Al, Sc, Ti, V, Cr, Mn, Fe, Co, Ni, Ga, Ge, Zr, Ta, Nb, B, Zn, Sn, W, and Mo,
x is a number of -1.4 or more and 1.5 or less,
y is a number of 0.0 or more and 3.0 or less,
z is a number of -5.0 or more and 5.2 or less,
a is a number of 0.1 or more and 10.4 or less, and
the ratio of the number of moles of A to the number of moles of Si is 1.4 or more and 3.7 or less.

6. The layered body according to claim 5, wherein the oxide represented by the formula (1) has a space group P6₃/m,
in the formula (1), A includes at least La, and
the oxide represented by the formula (1) has more Y atoms in a Wyckoff position 4f site than in a Wyckoff position 6h site out of sites occupied by La atoms.

7. The layered body according to any one of claims 1 to 6, further comprising an electrode.

8. The layered body according to claim 7, wherein the electrode contains:
one or more metals selected from the group consisting of Au, Ag, Pt, Pd, Rh, Ru, Os, and Ir;
a cation conductive carbonate; and
an oxide that contains: Li; and at least one selected from Ce and Sm.

9. Use of the layered body according to claim 8 as a gas sensor.

10. The layered body according to any one of claims 1 to 8, wherein the substrate has a through hole.

## Patentansprüche

1. Schichtkörper, umfassend:
- ein Substrat (11) und
- einen Oxidabschnitt (13), der ein Oxid enthält und auf dem Substrat angeordnet ist,
wobei das in dem Oxidabschnitt enthaltene Oxid Folgendes enthält: mindestens zwei oder mehr Seltenerdelemente; Silizium; und Sauerstoff,
wobei das in dem Oxidabschnitt enthaltene Oxid eine Apatit-Kristallstruktur aufweist,
**dadurch gekennzeichnet, dass** das in dem Oxidabschnitt enthaltene Oxid in einem Röntgendiffraktionsmuster ein Beugungsmaximum, das von der (004)-Ebene herrührt, bei einer Position von 2θ = 51,9° ± 0,9° zeigt,
und dass das Verhältnis des linearen Ausdehnungskoeffizienten des in dem Oxidabschnitt enthaltenen Oxids in Richtung der a-Achse zu dem linearen Ausdehnungskoeffizienten des Substrats ≥ 0,15 und ≤ 1,45 beträgt.

2. Schichtkörper nach Anspruch 1, wobei das Substrat Silizium enthält.

3. Schichtkörper nach Anspruch 2, wobei das Substrat mindestens eines ausgewählt aus der Gruppe bestehend aus Siliziumoxid und kristallinem Silizium enthält.

4. Schichtkörper nach einem der Ansprüche 1 bis 3, wobei das in dem Oxidabschnitt enthaltene Oxid Folgendes enthält: ein Lanthanoidelement; und mindestens eines oder mehrere ausgewählt aus Yttrium und Scandium.

5. Schichtkörper nach einem der Ansprüche 1 bis 4, wobei das in dem Oxidabschnitt enthaltene Oxid durch die Formel (1) dargestellt ist:
A_{9.3+x-a}Yₐ[Si_{6.0-y}M_{y}]O_{26.0+z}
- wobei A ein oder mehrere Elemente darstellt, ausgewählt aus der Gruppe bestehend aus La, Ce, Pr, Nd, Pm, Sm, Eu, Gd, Tb, Dy, Ho, Er, Yb, Lu, Be, Mg, Ca, Sr und Ba, und mindestens La umfasst,
- M ein oder mehrere Elemente darstellt, ausgewählt aus der Gruppe bestehend aus Mg, AI, Sc, Ti, V, Cr, Mn, Fe, Co, Ni, Ga, Ge, Zr, Ta, Nb, B, Zn, Sn, W und Mo,
- x eine Zahl von -1,4 oder mehr und 1,5 oder weniger ist,
- y eine Zahl von 0,0 oder mehr und 3,0 oder weniger ist,
- z eine Zahl von -5,0 oder mehr und 5,2 oder weniger ist,
- a eine Zahl von 0,1 oder mehr und 10,4 oder weniger ist, und
- das Verhältnis der Stoffmenge (Mol) von A zur Stoffmenge von Si 1,4 oder mehr und 3,7 oder weniger beträgt.

6. Schichtkörper nach Anspruch 5, wobei das durch die Formel (1) dargestellte Oxid die Raumgruppe P63/m aufweist, wobei in der Formel (1) A mindestens La umfasst, und
wobei das durch die Formel (1) dargestellte Oxid, unter den durch La-Atome besetzten Gitterplätzen, mehr Y-Atome auf einer Wyckoff-Position 4f als auf einer Wyckoff-Position 6h aufweist.

7. Schichtkörper nach einem der Ansprüche 1 bis 6, ferner umfassend eine Elektrode.

8. Schichtkörper nach Anspruch 7, wobei die Elektrode Folgendes enthält:
- ein oder mehrere Metalle ausgewählt aus der Gruppe bestehend aus Au, Ag, Pt, Pd, Rh, Ru, Os und Ir;
- ein kationenleitendes Carbonat; und
- ein Oxid, das Li enthält und mindestens eines ausgewählt aus Ce und Sm.

9. Verwendung des Schichtkörpers nach Anspruch 8 als Gassensor.

10. Schichtkörper nach einem der Ansprüche 1 bis 8, wobei das Substrat ein Durchgangsloch aufweist.

## Revendications

1. Corps multicouche comprenant :
- un substrat (11) ; et
- une portion d'oxyde (13) contenant un oxyde et positionnée sur le substrat,
dans lequel l'oxyde contenu dans ladite portion d'oxyde comprend : au moins deux ou plusieurs éléments des terres rares ; du silicium ; et de l'oxygène,
l'oxyde contenu dans ladite portion d'oxyde présente possède une structure cristalline de type apatite,
**caractérisé en ce que** l'oxide contenu dans la portion d'oxyde présente un pic de diffraction provenant d'un plan (004) à une position de 2θ = 51,9° ± 0,9° dans un diagramme de diffraction des rayons X, et,
le rapport du coefficient de dilatation linéaire de l'oxyde contenu dans ladite portion d'oxyde dans la direction de l'axe a par rapport au coefficient de dilatation linéaire du substrat est supérieur ou égal à 0,15 et inférieur ou égal à 1,45.

2. Corps multicouche selon la revendication 1, dans lequel le substrat contient du silicium.

3. Corps multicouche selon la revendication 2, dans lequel le substrat contient au moins un composé choisi parmi le groupe constitué d'un oxyde de silicium et de silicium cristallin.

4. Corps multicouche selon l'une quelconque des revendications 1 à 3, dans lequel l'oxyde contenu dans ladite portion d'oxyde comprend : un élément lanthanide ; et au moins un ou plusieurs choisis parmi l'yttrium et le scandium.

5. Corps multicouche selon l'une quelconque des revendications 1 à 4, dans lequel l'oxyde contenu dans ladite portion d'oxyde est représenté par la formule (1) : A_{9.3+x-a}Yₐ[Si_{6.0-y}M_{y}]O_{26.0+z},
- où A représente un ou plusieurs éléments choisis parmi le groupe constitué de La, Ce, Pr, Nd, Pm, Sm, Eu, Gd, Tb, Dy, Ho, Er, Yb, Lu, Be, Mg, Ca, Sr et Ba, et comprend au moins La,
- M représente un ou plusieurs éléments choisis parmi le groupe constitué de Mg, AI, Sc, Ti, V, Cr, Mn, Fe, Co, Ni, Ga, Ge, Zr, Ta, Nb, B, Zn, Sn, W et Mo,
- x est un nombre supérieur ou égal à -1,4 et inférieur ou égal à 1,5,
- y est un nombre supérieur ou égal à 0,0 et inférieur ou égal à 3,0,
- z est un nombre supérieur ou égal à -5,0 et inférieur ou égal à 5,2,
- a est un nombre supérieur ou égal à 0,1 et inférieur ou égal à 10,4, et
- le rapport du nombre de moles de A au nombre de moles de Si est supérieur ou égal à 1,4 et inférieur ou égal à 3,7.

6. Corps multicouche selon la revendication 5, dans lequel l'oxyde représenté par la formule (1) possède un groupe spatial P6₃/m,
dans la formule (1), A comprend au moins La, et
l'oxyde représenté par la formule (1) comporte davantage d'atomes de Y dans une position de Wyckoff 4f que dans une position de Wyckoff 6h parmi les sites occupés par les atomes de La.

7. Corps multicouche selon l'une quelconque des revendications 1 à 6, comprenant en outre une électrode.

8. Corps multicouche selon la revendication 7, dans lequel ladite électrode contient :
- un ou plusieurs métaux choisis parmi le groupe constitué de Au, Ag, Pt, Pd, Rh, Ru, Os et Ir ;
- un carbonate conducteur cationique ; et
- un oxyde contenant : du Li ; et au moins un choisi parmi Ce et Sm.

9. Utilisation du corps multicouche selon la revendication 8 en tant que capteur de gaz.

10. Corps multicouche selon l'une quelconque des revendications 1 à 8, dans lequel le substrat comporte un trou traversant.
